(19) Europäisches Patentamt
European Patent Office
Office européen des brevets

(11) Numéro de publication: **0 001 024**
**B1**

(12) **FASCICULE DE BREVET EUROPEEN**

(21) Numéro de dépôt: **78400072.1**

(22) Date de dépôt: **03.08.78**

(51) Int. Cl.³: **C 07 D 501/34,**
**A 61 K 31/545**

(54) **Forme cristalline du sel de sodium d'un dérivé oximiné de l'acide 7-amino thiazolyl acétamido céphalosporanique, son procédé de préparation et les compositions pharmaceutiques la renfermant**

(30) Priorité: **17.08.77 FR 7725142**

(43) Date de publication de la demande:
**07.03.79 Bulletin 79/05**

(45) Mention de la délivrance du brevet:
**21.01.81 Bulletin 81/03**

(84) Etats contractants désignés:
**BE CH DE FR GB LU NL SE**

(56) Documents cités:
**BE - A - 850 662**

(73) Titulaire: **ROUSSELL-UCLAF**
**102 route de Noisy Boîte postale no. 9**
**F - 93230 - Romainville (FR)**

(72) Inventeur: **Amiard, Gaston**
**7 bis, rue Hélène**
**F - 93130 - Noisy-Le-Sec (FR)**
**Bormann, Dieter**
**Johann-Strawss-Strasse 20**
**D - 6233 Kelkheim (Taunus) (DE)**
**Duerckheimer, Walter**
**Im Lerchenfeld 45**
**D - 6234 Hattersheim (DE)**
**Jolly, Jean**
**22, rue du Clos d'Orléans**
**F - 94120 - Fontenay-Sous-Bois (FR)**

(74) Mandataire: **Douetteau, Pierre, et al**
**c/o ROUSSELL-UCLAF 102, route de Noisy**
**Boîte postale 9**
**F - 93230 Romainville (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 001 024 B1

Courier Press, Leamington Spa, England.

Forme cristalline du sel de sodium d'un dérivé oximiné de l'acide 7-amino thiazolyl acétamido céphalosporanique, son procédé de préparation et les compositions pharmaceutiques la renfermant

Dans des demandes de brevets antérieures, et notamment dans le brevet belge 850.662, la Société demanderesse a décrit et revendiqué notamment:

— l'acide 3-acétoxyméthyl 7-/2-(2-amino 4-thiazolyl) 2-méthoxyimino acétamido/ ceph-3ème 4-carboxylique isomère syn et son sel de sodium.

De même des formes cristallines identifiables de ce sel de sodium ont été décrites dans ce brevet.

Ces produits sont doués d'une activité antibiotique remarquable sur les bactéries gram (+) et plus encore sur les bactéries gram (—).

La présente invention concerne une forme cristalline particulière du sel de sodium ci-dessus désigné, forme qui présente une excellente stabilité, particulièrement précieuse pour faciliter sa mise sous forme pharmaceutique.

La présente invention a ainsi pour objet la forme hydratée cristalline, dite forme D, du composé de formule:

Isomère *syn*,

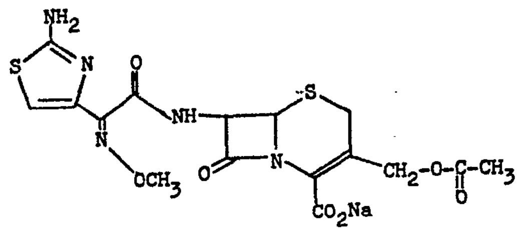

forme qui a le diagramme suivant de diffraction des poudres aux rayons X, diagramme obtenu avec la radiation K$\alpha$ du cuivre à une longueur d'onde de $\lambda = 1{,}54$ Å, diagramme dans lequel d représente les espacements inter-reticulaires et $I/I_1$ les intensités relatives

| d | $I/I_1$ |
|---|---|
| 9,3 | 0,95 |
| 8,8 | 0,09 |
| 6,5 | 0,07 |
| 6,46 | 0,06 |
| 6,10 | 0,13 |
| 5,30 | 0,25 |
| 5,12 | 0,05 |
| 5,03 | 0,04 |
| 4,57 | 0,04 |
| 4,40 | 0,17 |
| 4,20 | 0,29 |
| 3,96 | 0,70 |
| 3,88 | 0,18 |
| 3,76 | 0,34 |
| 3,6 | 1,00 |
| 3,41 | 0,15 |
| 3,27 | 0,09 |
| 3,19 | 0,11 |
| 3,13 | 0,13 |
| 3,10 | 0,13 |
| 3,03 | 0,15 |
| 2,97 | 0,06 |
| 2,78 | 0,07 |
| 2,72 | 0,04 |
| 2,69 | 0,04 |
| 2,60 | 0,19 |
| 2,50 | 0,11 |
| 2,44 | 0,05 |
| 2,40 | 0,08 |
| 2,32 | 0,09 |
| 2,25 | 0,05 |

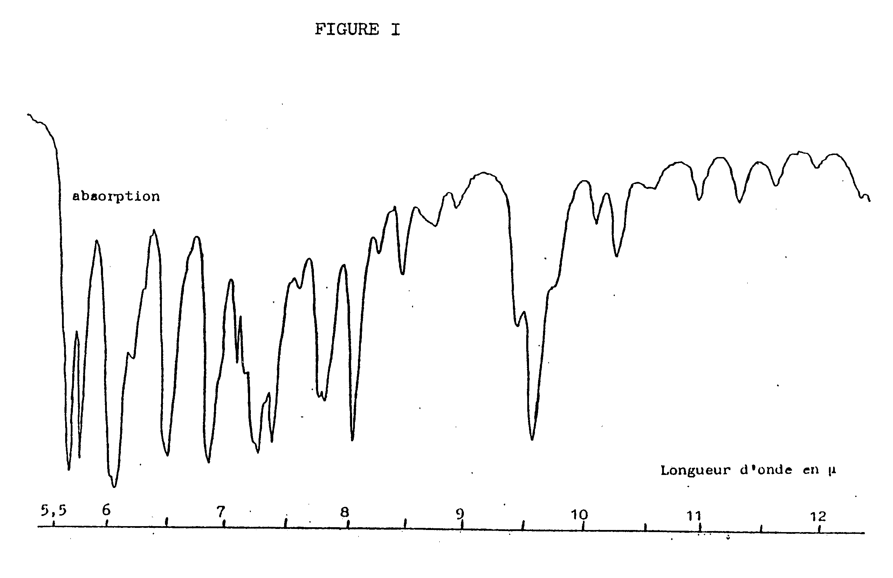

et qui possède dans le nujol le spectre infra-rouge représenté à la figure 1 annexée.

Le sel de sodium cristallisé de l'acide 3-acétoxyméthyl 7-/2-(2-amino 4-thiazolyl) 2-méthoxyimino acétamido/ceph-3-ème 4-carboxylique, isomère syn, a préalablement été obtenu sous d'autres formes cristallines identifiables. Cependant la forme cristalline dite forme "D" s'est révélée plus stable que les formes cristallines obtenues précédemment.

La nouvelle forme cristalline dite forme "D" est obtenue sous forme d'un solide blanc incolore.

Elle présente une faible affinité vis-à-vis de l'humidité atmosphérique et posséde de ce fait une stabilité très remarquable.

L'invention a également pour objet un procédé de préparation de la forme hydratée cristalline définie ci-dessus caractérisé en ce que:

A) — soit l'on traite l'acide de formule

isomère *syn*,

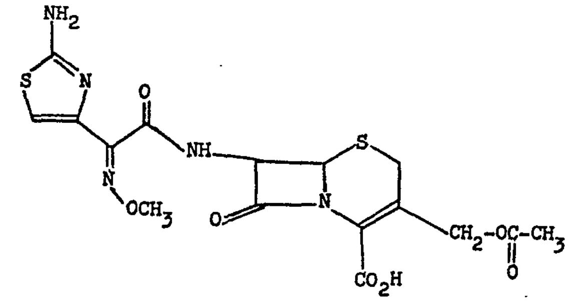

(I)

un hydrate, un solvat éthanolique, un solvat formique, ou un mélange d'hydrate et de solvat éthanolique ou formique de cet acide avec un sel de sodium de formule II:

Na X (II)

dans laquelle X représente l'anion d'un acide d'acidité comparable ou inférieure à celle de l'acide I et fait cristalliser le sel hydraté de formule III obtenu in situ

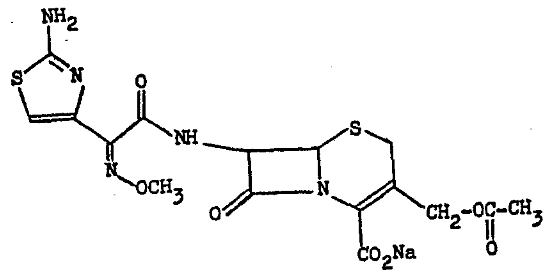

(III)

en présence si nécessaire d'un excès d'un solvant organique dans lequel le sel de sodium est pratiquement insoluble;

B) — soit l'on fait réagir l'acide de formule I, un hydrate, un solvat éthanolique, un solvat formique ou un mélange d'hydrate et de solvat éthanolique ou formique de cet acide, avec un sel de sodium d'un acide organique dans le méthanol, isole le sel de sodium de formule III cristallisé sous forme de solvat méthanolique, solvat que l'on transforme si désiré en produit de formule III ne contenant plus de méthanol et hydrate le produit de formule III ou son solvat méthanolique.

La préparation de l'acide de formule I, de son solvat formique et du mélange solvat formique — hydrate est décrite dans le brevet belge n° 850 662. Son solvat éthanolique peut être obtenu en traitant le formiate de cet acide par l'éthanol; l'hydrate de l'acide de formule I et le mélange d'hydrate et de solvat éthanolique peuvent être obtenus respectivement en laissant au contact de l'air humide l'acide de formule I et son solvat éthanolique. Comme sels de sodium de formule II, on peut citer des sels d'acides minéraux ou organiques.

Comme sels d'acides minéraux on peut mentionner par exemple le carbonate acide de sodium, le carbonate de sodium ou le phosphate tri-sodique.

Comme sels d'acides organiques on peut mentionner par exemple les sels de sodium d'acides carboxyliques aliphatiques linéaires ou ramifiés, saturés ou insaturés de 1 à 18 et de préférence de 2 à 10 atomes de carbone. Ces radicaux aliphatiques peuvent être interrompus par un ou plusieurs hétéroatomes tel que l'oxygène ou le soufre ou substitués par des radicaux aryle comme par exemple phényle, thiényle, furyle, par un ou plusieurs radicaux hydroxyle ou par un ou plusieurs atomes d'halogène tels que fluor, chlore ou brome, préférentiellement chlore, par un ou plusieurs radicaux

carboxyliques ou alkoxycarbonyles inférieurs, de préférence méthoxycarbonyle, éthoxycarbonyle ou propyloxycarbonyle, par un ou plusieurs radicaux aryloxy, de préférence phénoxy.

De plus on peut utiliser comme acides organiques des acides aromatiques suffisamment solubles comme par exemple des acides benzoïques substitués, de préférence par des radicauxalkyles inférieurs.

Comme exemples de tels acides organiques on peut mentionner — Les acides formique, acétique, acrylique, butyrique, adipique, isobutyrique, n-caproïque, isocaproïque, chloropropioniques, crotonique, phénylacétique, 2-thiénylacétique, 3-thiénylacétique, 4-éthylphénylacétique, glutarique, l'ester monoéthylique de l'acide adipique les acides hexanoïque, heptanoïque, décanoïque, oléïque, stéarique, palmitique, 3-hydroxypropionique, 3-méthoxypropionique, 3-méthylthiobutyrique, 4-chlorobutyrique, 4-phényl butyrique, 3-phénoxy butyrique, 4-éthyl benzoïque, 1-propyl benzoïque.

On utilise cependant de préférence un sel choisi dans le groupe formé par l'acétate de sodium, le 2-éthyl héxanoate de sodium et le diéthyl acétate de sodium et avantageusement, l'acétate de sodium.

Les sels de sodium des acides organiques mentionnés ci-dessus peuvent être utilisés tels quels ou peuvent être préparés in situ selon leur solubilité.

La préparation des sels de formule III peut être effectuée de différentes façons:

Selon la variante A énoncée ci-dessus, les sels de formule II sont placés dans une solution aqueuse, l'acide de formule I est ensuite introduit et le produit réactionnel est cristallisé en présence de solvants organiques dans lesquels le sel de céphalosporine est pratiquement insoluble. La formation du sel achevée, on mélange avec le solvant de précipitation suffisamment lentement afin que le sel de sodium ne commence pas à précipiter sous forme amorphe.

Des alcools et des cétones miscibles à l'eau se sont révélés être des solvants organiques pouvant être utiles dans cette réaction. Par exemple le méthanol, l'éthanol, le n-propanol, l'isopropanol ou l'acétone.

Un mode préférentiel d'exécution du procédé ci-dessus consiste à placer les sels de sodium de formule II dans un mélange de solvant organique et d'eau, à introduire l'acide de formule I et aprés que l'acide so soit dissous à faire cristalliser le sel de sodium, éventuellement en mélangeant avec un excès de solvant organique dans lequel le sel de sodium est presque insoluble, suffisament lentement afin que le sel de sodium ne commence pas à précipiter sous forme amorphe.

Le rapport entre l'eau et le solvant organique dans le mélange peut varier dans une certaine mesure; cependant un domaine d'environ 1 partie d'eau pour 5 parties de solvant à environ 1 partie d'eau pour 9 parties de solvant est préférable de manière à obtenir un produit cristallisé avec un haut rendement.

Dans le même but les sels de sodium de formule II sont utilisés de préférence en quantité au moins stoéchiométrique.

Dans certains cas, il est avantageux d'utiliser un excès du sel de sodium de formule II d'environ 10 à environ 200%, par example d'environ 20%.

La formation du sel de sodium peut être effectuée à des températures très variées. Cependant, de manière à avoir une manipulation qui n'altère pas les produits cherchés, par exemple afin d'éviter une cristallisation précoce, il est recommandé d'effectuer la formation du sel de sodium à une température égale ou inférieure à 0°C et de faire amorcer la cristallisation à une température supérieure à 0°C, plus spécialement à une température comprise entre environ 10 et 25°C. Cependant, le sel de sodium peut également être préparé avec un rendement comparable à température ambiante.

Les cristaux de sel de sodium de formule III préparés par le procédé de l'invention constituent la forme D stable.

Selon un mode préférentiel d'exécution du procédé, si l'on utilise le méthanol aqueux comme solvant dans la réaction de formation du sel de sodium, on dissout d'abord le sel de sodium, préférentiellement l'acétate de sodium, dans du méthanol aqueux maintenu à environ 0°C, introduit ensuite lentement l'acide I, un hydrate, un solvat éthanolique, un solvat formique ou un mélange d'hydrate et de solvat éthanolique ou formique de cet acid, puis agite la solution à environ 0°C. On peut alors, si désiré, procéder à une filtration stérilisante.

La phase de cristallisation de la forme D s'effectue préférentiellement en versant la solution obtenue dans un solvant dans lequel le sel est pratiquement insoluble.

Ce solvant peut être un solvant organique miscible à l'eau contenant de 0,5 à 10% d'eau, tel que le n-propanol, l'isopropanol, le tert-butanol, l'acétone ou de préférence l'éthanol.

Selon la variante B du procédé tel qu'indiqué ci-dessus, l'aide céphalosporanique I, sous forme non solvatée ou sous forme hydratée, solvatée avec l'éthanol ou l'acide formique ou sous forme d'un mélange d'hydrate et de solvat éthanolique ou formique, est transformé en sel de sodium dans le méthanol à l'aide du sel de sodium d'un acide organique aliphatique ou aromatique, tel que mentionné ci-dessus.

On utilise de préférence l'acétate de sodium, le 2-éthyl hexanoate de sodium ou le diéthylacétate de sodium.

Dans cette variante, un composé cristallin est obtenu sous la forme d'un solvat méthanolique. Ce solvat méthanolique peut être transformé en produit ne contenant plus de méthanol, par séchage intensif par exemple à environ 30 à 50°C sous vide. Les cristaux ainsi obtenus sont hygroscopiques.

Le solvat méthanolique, de même que le produit ne contenant plus de méthanol, sont transformés

par hydratation de manière à donner la formule D stable.

Cette hydratation peut être effectuée de diverses manières par exemple:

— ou bien, en maintenant le produit dans l'air ou dans un autre milieu tel que l'azote, ces milieux étant très humides,

— ou bien, le solvat méthanolique ou le produit ne contenant plus de méthanol peut être mis en suspension dans un solvant organique miscible à l'eau contenant de 1 à 10% d'eau. Ce solvant peut être une cétone telle que l'acétone ou un alcool tel que, par exemple, l'éthanol, le n-propanol, l'isopropanol ou le tert-butanol.

Une variante préférée consiste à mettre le sel de sodium en suspension dans l'éthanol contenant environ 1 á 10% d'eau et à agiter la suspension à température ambiante pendant environ 10 à 30 heures.

De cette manière, le solvat méthanolique, de même que le composé ne contenant plus de méthanol, peuvent être transformés de manière à donner la phase cristalline stable dite forme "D".

La teneur en eau de la forme cristalline D dépend de l'humidité du milieu et peut aller jusqu'à 15% sans modification de la structure cristalline.

Par séchage, particulièrement sous vide à température ambiante, la teneur en eau peut être réduite au domaine préféré d'environ 3% à environ 6%.

L'invention a également pour objet un procédé de préparation de la forme hydratée cristalline définie ci-dessus, caractérisé en ce que l'on traite l'acide de formule I sous forme non solvatée, sous forme hydratée, sous forme solvatée avec l'éthanol ou l'acide formique, ou sous forme d'un mélange d'hydrate et de solvat éthanolique ou formique, par un sel de sodium d'un acide organique dans le méthanol, isole le solvat méthanolique cristallin du sel de sodium obtenu, met en suspension ce solvat méthanolique dans un solvant miscible à l'eau et contenant une faible quantité d'eau, laisse en suspension, puis isole la forme cristalline D cherchée.

Dans un mode préférentiel d'exécution du procédé ci-dessus le sel de sodium d'un acide organique que l'on utilise est choisi dans le groupe formé par l'acétate de sodium, le diéthylacétate de sodium et l'éthyl hexanoate de sodium.

Dans une variante d'exécution particulièrement avantageuse du procédé qui vient d'être décrit, le solvant contenant une faible quantité d'eau dans lequel on met en suspension le solvat méthanolique pour obtenir la forme cristalline D est de l'éthanol contenant de 1 à 10% d'eau.

Les conditions de mise en oeuvre des procédés permettent de réaliser des opérations stériles.

Comme les autres formes du sel de sodium de l'acide 3-acétoxyméthyl 7-/2-(2-amino 4-thiazolyl) 2-méthoxyimino acétamido/ cèph 3-ème 4-carboxylique, la forme dite forme "D" possède une très bonne activité antibiotique d'une part sur les bactéries gram (+), telles que les staphylocoques, les streptocoques et notamment sur les staphylocoques pénicillino-résistants et, d'autre part, sur les bactéries gram (—) notamment sur les bactéries coliformes, les Klebsiella, les Proteus, et les Salmonella.

Ces propriétés rendent aptes ledit produit à être utilisé comme médicament dans le traitement des maladies à germes sensibles et plus particulièrement dans celui des staphylococcies, telles que septicémies à staphylocoques, staphylococcies malignes de la face ou cutanées, pyodermites, plaies septiques ou suppurantes, anthrax, phlegmons, érésipèles, staphylococcies aiguës primitives ou post-grippales, bronchopneumonies, suppurations pulmonaires.

Ce produit peut également être utilisé comme médicament dans le traitement des colibacilloses et infections associées, dans les infections à Proteus, à Klebsiella et à Salmonella et dans d'autres affections provoquées par des bactéries à gram (—).

Ces Propriétés justifient l'utilisation en thérapeutique, à titre de médicament et notamment de médicament antibiotique, du sel de sodium sous forme hydratée cristalline, dite forme D, de l'acide 3-acétoxyméthyl 7-/2-(2-amino 4-thiazolyl) 2-méthoxyimino acétamido/ céph-3-ème 4-carboxylique, isomère *syn*.

L'invention s'étend aux compositions pharmaceutiques renfermant, comme principe actif, le médicament défini ci-dessus.

Ces compositions peuvent être administrées par voie buccale, rectale, parentérale ou par voie locale en application topique sur la peau et les muqueuses.

Elles peuvent être solides ou liquides et se présenter sous les formes pharmaceutiques couramment utilisées en médecine humaine, comme, par exemple, les comprimés simples ou dragéifiés, les gélules, les granulés, les suppositoires, les préparations injectables, les pommades, les crèmes, les gels; elles sont préparées selon les méthodes usuelles.

Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La dose administrée est variable selon l'affection traitée, le sujet en cause, la voie d'administration et le produit considéré. Elle peut être, par exemple, comprise entre 0,250 g et 4 g par jour, par voie

orale, chez l'homme ou encore comprise entre 0,500 g et 1 g, trois fois par jour, par voie intramusculaire.

Les exemples suivants illustrent l'invention sans toutefois la limiter.

Exemple 1

Sel de sodium cristallisé sous la forme "D" de l'acide 3-acétoxyméthyl 7-/2(2 amino 4-thiazolyl) 2-méthoxyimino acétamido/ céph-3ème 4-carboxylique isomère *syn*.

*STADE A* — Solvat méthanolique cristallisé du sel de sodium de l'acide 3-acétoxyméthyl 7-/2(2-amino 4-thiazolyl) 2-méthoxyimino acétamido/céph 3ème 4-carboxylique isomère *syn*.

On introduit dans 12 1 de méthanol pur anhydre, 432 g d'acétate de sodium anhydre. Après dissolution on ajoute en une fois 3 000 g d'acide 3-acétoxyméthyl 7-/2-(2-amino 4-thiazolyl) 2 méthoxyimino acétamido/céph 3ème 4-carboxylique isomère *syn* contenant 5% d'éthanol et 0,9% d'eau. On agite à 18—20°C sous azote et obtient après 5 minutes une solution presque totale. Cette solution est envoyée dans le réacteur sous pression d'azote(1,5 à 2 bars).

A la solution conservée à 15—20°C on ajoute sous agitation très *efficace* et atmosphère d'azote et, en une seule fois, à 18—20°C 864 g d'acétate de sodium anhydre dans 4,5 l de méthanol. On obtient une solution limpide qui est maintenue sous agitation et azote à 18—20°C. On prélève aussitôt 10 $cm^3$ de la solution réactionnelle, provoque la cristallisation par grattage en réintroduit la suspension ainsi obtenue dans le milieu. La cristallisation commence après 5 minutes environ. On maintient 4 heures à 18—20°C sous agitation rapide et atmosphère d'azote. La cristallisation augmente progressivement. La suspension est ensuite refroidie à 0, +2°C puis agitée 2 heures. On transvase et essore. On lave par empâtage avec 3 fois 1 l de méthanol pur anhydre à 0, +2°C puis 1 fois avec 1,5 l. On seche environ 48 heures à 20—22°C sous vide et obtient 2 630 g de produit attendu.

$H_2O$ (par Fischer) = 1%

Méthanol par chromatographie en phase vapeur = 8%

Le produit obtenu est passé au tamis pour l'homogénéiser.

*STADE B* — Sel de sodium cristallisé sous la forme "D" de l'acide 3-acétoxyméthyl 7-/2 (2 amino 4-thiazolyl) 2 méthoxyimino acétamido/céph 3 ème 4-carboxylique isomère *syn*

100 g du produit tamisé obtenu au stade précédent sont placés dans 800 cm3 d'éthanol à 5% d'eau. On agite 15 à 20 heures à 20°C, ± 2°C. On essore, lave avec 100 cm3 d'éthanol à 5% d'eau, sèche en étuve sous vide à 20—25°C et obtient 99 g de produit attendu. (poids non corrigé)

$H_2O$ = 5,3%

EtOH < 0,2%

MeOH < 0,2%

Ce produit présente les spectres Infra-Rouge et de diffraction X mentionnés dans la description.

L'acide 3-acétoxyméthyl 7-/2 (2 amino 4-thiazolyl)2 méthoxyimino acétamido/céph 3ème 4-carboxylique isomère *syn* utilisé au départ de l'exemple à été décrit dans le brevet belge n° 850 662.

Exemple 2

Sel de sodium cristallisé sous la forme "D" de l'acide 3-acétoxyméthyl 7-/2(2-amino 4-thiazolyl)2-méthoxyimino acétamido/ceph 3éme 4-carboxylique isomère *syn*.

On ajoute à 20°C et sous agitation, 50 cm3 d'éthanol à 98% à une solution de 6 g d'acétate de sodium dans 20 cm3 d'eau et, en poursuivant l'agitation, ajoute 25 g de solvat éthanolique de l'acide 3-acétoxyméthyl 7-/2-(2 amino 4-thiazolyl)2-méthoxyimino acétamido/céph 3ème 4-carboxylique isomère *syn*.

L'acide passe en solution, cette solution est refroidie à 0°C. On ajoute goutte à goutte en agitant, 50 cm3 d'éthanol à 98%. La solution est ensuite filtrée après addition de charbon actif et le filtrat est chauffé à 20°C. La cristallisation s'amorce spontanément peu après. La filtration par aspiration est effectuée à température ambiante après 16 heures. Le produit obtenu est d'abord lavé à l'aide d'un mélange eau-éthanol dans la proportion 1 à 7 puis à l'éthanol, enfin à l'éther.

Les cristaux incolores obtenus sont séchés sous vide poussé:

$H_2O$ (par Fischer) : 4,8%

Le spectre de diffraction X et le spectre infra-rouge dans le nujol confirment qu'il s'agit bien de la forme D.

Exemple 3

Sel de sodium cristallisé sous la forme "D" de l'acide 3-acétoxyméthyl 7-/2-(2-amino 4-thiazolyl) 2-méthoxyimino acétamido/céph 3ème 4-carboxylique isomère *syn*.

On utilise le même mode opératoire que celui décrit à l'exemple 2 à cette différence près que l'on emploie le sel de sodium de l'acide 2-éthylhexanoïque au lieu de l'acétate de sodium.

En opérant dans des conditions absolument identiques on obtient des cristaux que l'on sèche sous vide poussé. Le spectre infra-rouge dans le nujol confirme l'obtention de la forme "D" stable.

Exemple 4

Sel de sodium cristallisé sous la forme "D" de l'acide 3-acétoxyméthyl 7-/2-(2-amino 4-thiazolyl)2-méthoxyimino acétamido/céph 3ème 4-carboxylique isomère *syn.*

5,01 g de solvat éthanolique de l'acide 3-acétoxyméthyl 7-/2-(2-amino 4-thiazolyl)2-méthoxyimino acétamido/céph 3ème 4-carboxylique, isomère *syn*, sont ajoutés à une solution de 2,2 g de diéthylacétate de sodium dans un mélange de 4 cm3 d'eau et 20 cm3 d'éthanol à 98%. L'acid céphem carboxylique passe complétement en solution. On ajoute 0,5 g de charbon actif au mélange réactionnel, agite 5 minutes et filtre. Le filtrat est agité à température ambiante et la cristallisation débute spontanément après environ 1 heure.

Aprés une nuit à température ambiante, les cristaux précipités sont isolés, lavés d'abord avec un mélange d'éthanol et d'eau dans la proportion de 7 à 1, puis à l'éthanol et enfin à l'éther et séchés sous vide poussé pendant 2 heures.

On obtient le sel de sodium sous la forme D cherchée. Ceci est confirmé par le spectre infra-rouge dans le nujol.

Exemple 5

Sel de sodium cristallisé sous la forme D de l'acide 3-acétoxyméthyl 7-/2 (2-amino 4-thiazolyl) 2-méthoxyimino acétamido/céph 3ème 4-carboxylique isomère *syn.*

4,55 g d'acide 3-acétoxyméthyl 7-/2-(2-amino 4-thiazolyl) 2-méthoxyimino acétamido/céph 3ème 4-carboxylique, isomère *syn*, sont introduits dans une solution de 0,85 g de carbonate acide de sodium dans 5 cm3 d'eau. On abtient une solution visqueuse à laquelle on ajoute ensuite goutte à goutte 10 cm3 d'éthanol à 98% et filtre. On ajoute ensuite goutte à goutte à la solution ainsi obtenue, 20 cm3 d'éthanol à température ambiante et sous agitation.

On laisse ensuite cristalliser. Lorsque la cristallisation est achevée les cristaux incolores sont isolés, lavés par un mélange éthanol et eau dans la proportion de 7 à 1 et séchés.

Le spectre de diffraction aux rayons X et le spectre infra-rouge dans le nujol prouvent l'identité du se de sodium ainsi obtenu avec celui de l'exemple 1.

Exemple 6

Sel de sodium cristallisé sous la forme D de l'acide 3-acétoxyméthyl 7-/2-(2-amino 4-thiazolyl) 2-méthoxyimino acétamido/céph 3ème 4-carboxylique isomère *syn.*

15 g d'acide 3-acétoxyméthyl 7-/2-(2-amino 4-thiazolyl) 2-méthoxyimino acétamido/céph 3ème 4-carboxylique, isomère *syn*, sont introduits et dissous à —15°C dans une solution de 5 g d'acétate de sodium dans 100 cm3 de méthanol. On ajoute 3 g de charbon actif à la solution puis filtre et chauffe à 20°C.

Les cristaux de solvat méthanolique du sel de sodium de l'acide 3-acétoxyméthyl 7-/2-(2-amino 4-thiazolyl)2-méthoxyimino acétamido/céph 3ème 4-carboxylique isomère *syn* identiques à ceux obtenus au stade A de l'exemple 1 sont isolés.

A) — Une partie de cristaux ainsi obtenus est séchée sous vide poussé, le méthanol est éliminé et les cristaux ne contenant plus de méthanol sont maintenus une nuit dans une atmosphère saturée en vapeur d'eau.

Les cristaux incolores obtenus sont séchés à température ambiante sous vide.

Les cristaux ainsi obtenus contiennent 5,2% d'eau (selon la méthode de Fischer).

Les spectres de diffraction aux rayons X et d'infra-rouge dans le nujol confirment l'identité avec le produit obtenu à l'exemple 1. Il s'agit donc bien de la forme D.

B) — Une autre partie des cristaux est maintenue, sans autre traitement préalable, une nuit sous atmosphère saturée de vapeur d'eau. Les cristaux sont isolés et séchés deux heures sous vide à température ambiante.

La méthode de Fischer indique la présence de 4,9% d'eau. Les spectres de diffraction aux rayons X et d'infra-rouge dans le nujol prouvent l'identité avec le produit obtenu à l'exemple 1. Il s'agit donc bien de la forme D.

Exemple 7

Sel de sodium cristallisé sous la forme D de l'acide 3-acétoxyméthyl 7-/2-(2-amino 4-thiazolyl) 2-méthoxyimino acétamido/céph 3ème 4-carboxylique isomère *syn.*

5,01 g de solvat éthanolique de l'acide 3-acétoxyméthyl 7-/2 (2-amino 4-thiazolyl) 2-méthoxyimino acétamido/céph 3ème 4-carboxylique, isomère *syn,* sont ajoutés à température ambiante à une solution de 1,2 g d'acétate de sodium dans 10 cm3 d'eau et 15 cm3 d'isopropanol. On traite ensuite la solution par 0,5 g de charbon actif et filtre. On ajoute goutte à goutte et sous agitation 25 cm3 d'isopropanol au filtrat.

Les cristaux sont isolés après 5 heures et demie, lavés avec un mélange d'isopropanol et d'eau et enfin séchés pendant 3 heures à température ambiante et sous vide poussé.

Les cristaux ainsi obtenu sont d'après le spectre infra-rouge identiques à ceux de l'exemple 1. Il s'agit donc de la forme D.

Exemple 8

Sel de sodium cristallisé sous la forme D de l'acide 3-acétoxyméthyl 7-/-(2-amino 4-thiazolyl) 2-méthoxyimino acétamido/céph 3ème 4-carboxylique isomère *syn*.

On utilise le même mode opératoire que celui décrit à l'exemple 7 à cette différence près que l'on emploie de l'acétone au lieu de l'isopropanol.

Selon le spectre infra-rouge dans le nujol, les cristaux obtenus sont complètement identiques à ceux décrits à l'exemple 1. Il s'agit donc de la forme D.

Exemple 9

Sel de sodium cristallisé sous la forme D de l'acide 3-acétoxyméthyl 7-/2-(2-amino 4-thiazolyl) 2-méthoxyimino acétamido/céph-3-ème 4-carboxylique, isomère *syn*.

On mélange 175 cm3 de méthanol pur anhydre, 25 cm3 d'eau déminéralisée et 21,6 g d'acétate de sodium anhydre. On amène la solution à 0°, +2°C sous agitation et sous atmosphère d'azote.

En maintenant la température à 0°, +2°C, on introduit 108,7 g d'acide 3-acétoxyméthyl 7-/2-(2-amino 4-thiazolyl) 2-méthoxyimino acétamido/céph-3-ème 4-carboxylique, isomère *syn*, contenant environ 8% d'éthanol et 0,5% d'eau.

Après dissolution de l'acide, on agite sous azote à 0°, +2°C, puis la solution est passée sur un filtre stérilisant calorifugé et est reçue à 0°, +2°C sous azote dans une enceinte stérile.

On rince avec deux fois 25 cm3 de méthanol pur anhydre. La solution stérile maintenue sous azote à 0°C, +2°C est précipitée régulièrement en une heure sous agitation, sous azote dans 500 cm3 d'éthanol à 2% d'eau et à une température de 18—20°C.

La suspension obtenue est laissée sous agitation pendant plusieurs heures sous azote.

On essore. Le produit est empâté successivement avec 100, puis 200 cm3 d'éthanol à 5% d'eau. On termine par un lavage avec 50 cm3 d'éthanol à 5% d'eau. On obtient le produit humide que l'on sèche sous vide à 20—25°C. On obtient ainsi 96,5 g de produit attendu.

$H_2O$ = 4%

EtOH $\simeq$ 0,4%.

Ce produit présente le spectre infrarouge de la forme D.

Exemple 10

Sel de sodium cristallisé sous la forme D de l'acide 3-acétoxyméthyl 7-/2-(2-amino 4-thiazolyl) 2-méthoxyimino acétamido/ceph 3-ème-4-carboxylique, isomère *syn*.

A une solution de 2 g d'acétate de sodium dans un mélange de 7,5 cm3 d'eau et 20 cm3 d'éthanol absolu, on ajoute 10 g d'acide 3-acétoxyméthyl 7-/2-(2-amino 4-thiazolyl) 2-méthoxyimino acétamido/céph-3-éme 4-carboxylique, isomère *syn*, contenant environ 7,2% d'éthanol.

On agite 15 minutes à température ambiante, traite par 1,5g de charbon actif et filtre.

On lave deux fois le charbon résiduel par 5 cm3 d'un mélange éthanol — eau (9—1).

Le filtrat est versé goutte à goutte à température ambiante sous agitation et en 50 minutes, dans 80 cm3 d'éthanol. Le sel de sodium cristallise. On agite le mélange réactionnel pendant 2 heures à 0°C, essore, lave les cristaux à l'éthanol et les sèche sous vide. On obtient 8,45 g de produit attendu. $H_2O$ : 5,7%.

Exemple 11

Sel de sodium cristallisé sous la forme D de l'acide 3-acétoxyméthyl 7-/2-(2-amino 4-thiazolyl) 2-méthoxyimino acétamido/ceph-3 ème 4-carboxylique, isomère *syn*.

On opère comme indiqué à l'exemple précédent jusqu'au traitement au charbon actif.

Le filtrat est versé goutte à goutte à température ambiante sous agitation et en 2 heures, dans 80 cm3 d'isopropanol. Après 2 heures à température ambiante, on agite 2 heures à 0°C, essore, les cristaux, les lave à l'éthanol et les sèche sous vide poussé. On obtient 8,80 g de produit attendu. $H_2O$ : 0,74%.

Exemple 12

Sel de sodium cristallisé sous la forme D de l'acide 3-acétoxyméthyl 7-/2-(2-amino 4-thiazolyl) 2-méthoxyimino acétamido/ceph-3-ème 4-carboxylique, isomère *syn*.

A une solution de 3,5 g d'acétate de sodium dans 5 cm3 d'eau et 300 cm3 d'éthanol, on ajoute à température ambiante 10 g de formiate de l'acide 3-acétoxyméthyl 7-/2-(2-amino 4-thiazolyl) 2-méthoxyimino acétamido/ceph-3-éme 4-carboxylique, isomère *syn*. Quand la dissolution est terminée, on filtre et verse le filtrat goutte à goutte à température ambiante en une heure et sous agitation, dans 160 cm3 d'éthanol absolu. On agite pendant 3 heures à température ambiante, puis maintient à 0°C pendant 16 heures. On essore les cristaux, les lave à l'éthanol et les sèche sous vide poussé. On obtient

7,75 g de produit attendu.

$H_2O$ : 0,95%.

Le solvat avec l'acide formique de l'acide de départ ci-dessus est décrit dans le brevet beige n° 850 662.

Exemple 13

On a réalisé une préparation pour injection de formule

| | | |
|---|---|---|
| — sel de sodium cristallisé, sous la forme "D", de l'acide 3-acétoxyméthyl 7-/2 (2-amino 4-thiazolyl) 2 méthoxyimino acétamido/ceph 3ème 4-carboxylique, isomère *syn* | | 500 mg |
| —Excipient aqueux stérile | q.s.p. | 5cm3 |

On a réalisé des gélules répondant à la formule:

| | |
|---|---|
| — Sel de sodium cristallisé sous la forme "D" de l'acide 3-acétoxyméthyl 7-/2 (2-amino 4-thiazolyl)2 méthoxyimino acétamido/céph 3éme 4-carboxylique, isomère *syn* | 250 mg |
| — Excipient q.s pour une gélule terminée à | 400 mg |

Etude pharmacologique du produit de l'invention Activité in vitro:— *Méthode des dilutions en milieu liquide:*

On prépare une série de tubes dans lesquels on répartit une même quantité de milieu nutritif stérile. On distrubue dans chaque tube des quantités croissantes du produit à étudier, puis chaque tube est ensemencé avec une souche bactérienne.

Après incubation de vingt-quatre ou quarante-huit heures à l'étuve à 37°C, l'inhibition de la croissance est appréciée par transillumination ce qui permet de déterminer les concentrations minimales inhibitrices (C.M.I.) exprimées en $\mu g$/cm3.

Les résultats suivants ont été obtenus.

| SOUCHES | Produit de l'exemple 1 | C.M.I en µg/ml |
|---|---|---|
| | 24 H | 48 H |
| Staphylococcus aureus I H 005 | 2 | 3 |
| Staphylococcus aureus I H 38 | 1 | 2 |
| Staphylococcus aureus I H 41 | 1 | 2 |
| Staphylococcus aureus I H 47 | 2 | 2 |
| Escherichia coli 7881 | 0,1 | 0,1 |
| Escherichia Coli T 55 4376 | 0,1 | 0,1 |
| Escherichia Coli T 55 4076 | 0,05 | 0,05 |
| Escherichia Coli A 223 | 0,1 | 0,1 |
| Klebsiella Pneumoniae COc 229 | 0,1 | 0,1 |
| Klebsiella Pneumoniae COc 238 | 0,2 | 0,2 |
| Proteus Mirabilis 3477 | 0,05 | 0,05 |
| Proteus Mirabilis 1816 | 0,05 | 0,05 |
| Proteus Mirabilis 9048 | 0,02 | 0,02 |
| Proteus Vulgaris Co 1 | 0,2 | 0,4 |
| Proteus Vulgaris Co 4 | 0,6 | 0,6 |
| Proteus Vulgaris Du 27 | ⩽0.02 | ⩽0.02 |
| Salmonella typhimurium 6478 | 0,1 | 0,1 |

Essais de stabilité du produit de l'exemple 1.

Le produit est placé dans un flacon contenant de l'air non stérile et fermé par un bouchon de caoutchouc.

On obtient les résultats suivants exprimés en pourcentage d'activité résiduelle.

| Temps 0 | 80°C | | 50°C | |
|---|---|---|---|---|
| | 160 H | 2 semaines | 1,5 mois | 3 mois |
| 97 | 89,5 | 81,5 | 92,5 | 92 |

**Revendications**

1. Forme hydratée cristalline, dite forme "D", du composé de formule:

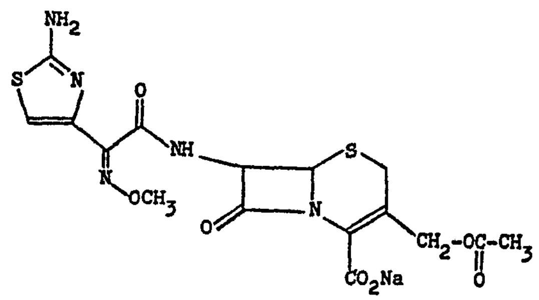

Isomère *syn*,

forme qui a le diagramme suivant de diffraction des poudres aux rayons X, diagramme obtenu avec la radiation k$\alpha$ du cuivre à une longueur d'onde de $\lambda = 1{,}54$ Å, diagramme dans lequel d représente les espacements inter-reticulaires et $I/I_1$ les intensités relatives

| d | $I/I_1$ |
|---|---|
| 9,3 | 0,95 |
| 8,8 | 0,09 |
| 6,5 | 0,07 |
| 6,46 | 0,06 |
| 6,10 | 0,13 |
| 5,30 | 0,25 |
| 5,12 | 0,05 |
| 5,03 | 0,04 |
| 4,57 | 0,04 |
| 4,40 | 0,17 |
| 4,20 | 0,29 |
| 3,96 | 0,70 |
| 3,88 | 0,18 |
| 3,76 | 0,34 |
| 3,6 | 1,00 |
| 3,41 | 0,15 |
| 3,27 | 0,09 |
| 3,19 | 0,11 |
| 3,13 | 0,13 |
| 3,10 | 0,13 |
| 3,03 | 0,15 |
| 2,97 | 0,06 |
| 2,78 | 0,07 |
| 2,72 | 0,04 |
| 2,69 | 0,04 |
| 2,60 | 0,19 |
| 2,50 | 0,11 |
| 2,44 | 0,05 |
| 2,40 | 0,08 |
| 2,32 | 0,09 |
| 2,25 | 0,05 |

et qui possède dans le nujol le spectre infra-rouge représenté à la figure 1 annexée.

2. Procédé de préparation de la forme hydratée cristalline définie à la revendication 1 caractérisé en ce que:

A) — soit l'on traite l'acide de formule 1:

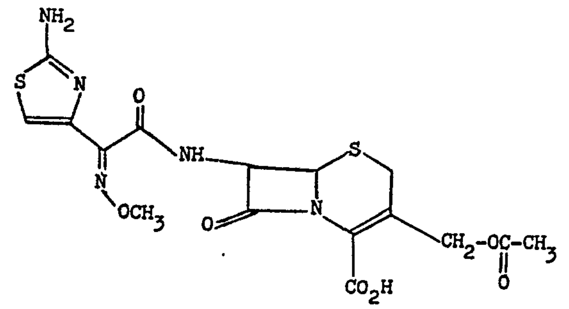

isomère *syn*, (I)

un hydrate, un solvat éthanolique, un solvat formique ou un mélange d'hydrate et de solvat éthanolique ou formique de cet acide avec un sel de sodium de formule II:

Na X (II)

dans laquelle X représente l'anion d'un acide d'acidité comparable ou inférieure à celle de l'acide I et fait cristalliser le sel hydraté de formule III obtenu in situ

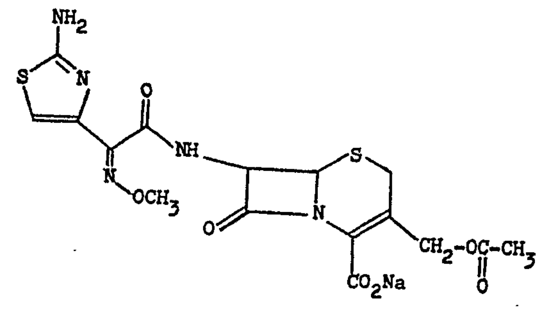

(III)

en présence si nécessaire d'un excès d'un solvant organique dans lequel le sel de sodium est pratiquement insoluble;

B) — soit l'on fait réagir l'acide de formule (I) un hydrate, un solvat éthanolique ou formique ou un mélange d'hydrate et de solvat éthanolique ou formique de cet acide, avec un sel de sodium d'un acide organique dans le méthanol, isole le sel de sodium de formule (III) cristallisé sous forme de solvat méthanolique, solvat que l'on transforme, si désiré, en produit de formule (III) ne contenant plus de méthanol et hydrate le produit de formule (III) ou son solvat méthanolique.

3. Procédé selon la revendication 2, caractérisé en ce que l'on effectue le traitement de l'acide I, de l'hydrate, du solvat éthanolique, du solvat formique ou du mélange de l'hydrate et du solvat éthanolique ou formique de cet acide par le sel de sodium II dans le méthanol aqueux, à une température d'environ 0°C.

4. Procédé selon la revendication 2 de préparation du sel hydraté cristallin défini à la revendication 1, caractérisé en ce que l'on traite l'acide de formule (I), un hydrate, un solvat éthanolique ou formique ou un mélange d'hydrate et de solvat éthanolique ou formique de cet acide par un sel de sodium d'un acide organique dans le méthanol, isole la solvat méthanolique cristallin du sel de sodium obtenu, met en suspension ce solvat méthanolique dans un solvant miscible à l'eau et contenant une faible quantité d'eau, laisse en suspension, puis isole la forme cristalline D cherchée.

5. Procédé selon l'une quelconque des revendications 2 à 4, caractérisé en ce que le sel de sodium d'un acide organique que l'on utilise est choisi dans le groupe formé par l'acétate de sodium, l'éthyl hexanoate de sodium et le diéthylacétate de sodium.

6. Procédé selon l'une quelconque des revendications 3, 4 ou 5, caractérisé en ce que le solvant dans lequel on met en suspension le solvat méthanolique ou avec lequel on mélange la solution méthanolique du sel de sodium de l'acide I pour obtenir la forme cristalline D, est de l'éthanol contenant de 0,5 à 10% d'eau.

7. Compositions pharmaceutiques, caractérisées en ce qu'elles renferment, à titre de principe actif, le sel de sodium sous forme hydratée cristalline, dite forme D, de l'acide 3-acétoxyméthyl 7-/2-(2-amino 4-thiazolyl) 2-méthoxyimino acétamido/ceph-3-ème 4 carboxylique, isomère *syn*.

8. A titre de médicament, le sel de sodium sous forme hydratée cristalline, dite forme D, de l'acide

3-acétoxyméthyl 7-/2-(2-amino 4-thiazolyl) 2-méthoxyimino acétamido/céph-3-ème 4-carboxylique, isomère *syn*.

## Patentansprüche

1. Als Form "D" bezeichnete kristalline hydratisierte Form der Verbindung der Formel:

*syn*-Isomeres

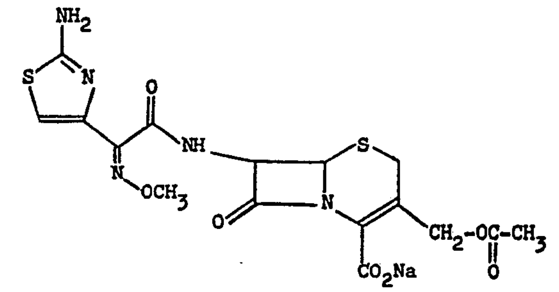

wobei diese Form das folgende Röntgenbeugungspulverdiagramm besitzt, erhalten durch Kupfer-K$\alpha$-Bestrahlung bei einer Wellenlänge von $\lambda = 1{,}54$ Å, in dem d die Gitterabstände bedeutet und $I/I_1$ die relativen Intensitäten darstellt:

| d | I/$I_1$ |
|---|---|
| 9,3 | 0,95 |
| 8,8 | 0,09 |
| 6,5 | 0,07 |
| 6,46 | 0,06 |
| 6,10 | 0,13 |
| 5,30 | 0,25 |
| 5,12 | 0,05 |
| 5,03 | 0,04 |
| 4,57 | 0,04 |
| 4,40 | 0,17 |
| 4,20 | 0,29 |
| 3,96 | 0,70 |
| 3,88 | 0,18 |
| 3,76 | 0,34 |
| 3,6 | 1,00 |
| 3,41 | 0,15 |
| 3,27 | 0,09 |
| 3,19 | 0,11 |
| 3,13 | 0,13 |
| 3,10 | 0,13 |
| 3,03 | 0,15 |
| 2,97 | 0,06 |
| 2,78 | 0,07 |
| 2,72 | 0,04 |
| 2,69 | 0,04 |
| 2,60 | 0,19 |
| 2,50 | 0,11 |
| 2,44 | 0,05 |
| 2,40 | 0,08 |
| 2,32 | 0,09 |
| 2,25 | 0,05 |

und in Nujol das in der beigefügten Fig. 1 gezeigte Infrarot-spektrum aufweist.

2. Verfahren zur Herstellung der in Anspruch 1 definierten kristallinen hydratisierten Form, dadurch gemennzeichnet, daß man

A) entweder die Säure der Formel I:

syn-Isomeres

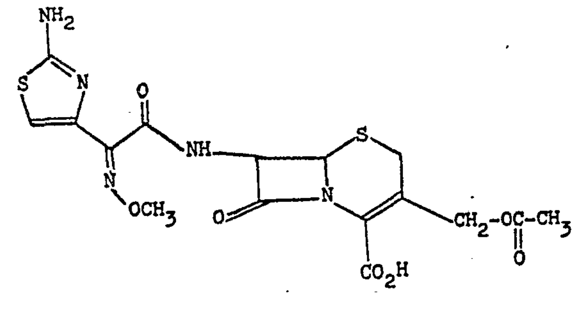

(I)

ein Hydrat, ein Äthanolsolvat, ein Ameisensäuresolvat oder ein Gemisch des Hydrats und des Äthanol- oder Ameisensäuresolvats dieser Säure mit einem Natriumsalz der Formel II:

Na X (II)

in der X das Anion einer Säure mit einer Acidität vergleichbar derjenigen der Säure I oder geringer als derjenigen der Säure I behandelt und das erhaltene hydratisierte Salz der Formel III:

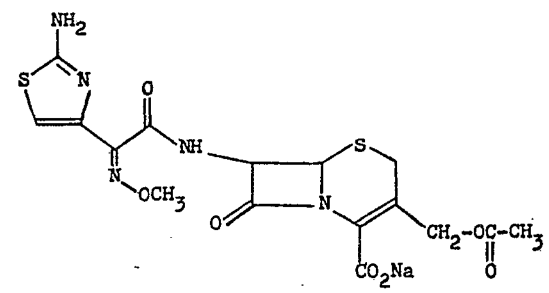

(III)

in situ, gegebenenfalls in Anwesenheit eines Überschusses eines organischen Lösungsmittels, in dem das Natriumsalz praktisch unlöslich ist, zur Kristallisation bringt;

B) oder die Säure der Formel I, ein Hydrat, ein Äthanol- oder Ameisensäuresolvat oder ein Gemisch des Hydrats und des Äthanol- oder Ameisensäuresolvats dieser Säure mit einem Natriumsalz einer organischen Säure in Methanol umsetzt, das in Form des methanolischen Solvats kristallisierte Natriumsalz der Formel III isoliert, welches Solvat man gewünschtenfalls in ein Produkt der Formel III, das kein Methanol mehr enthält, überführt und das Produkt der Formel III oder sein Methanolsolvat hydratisiert.

3. Verfahren gemäß Anspruch 2, dadurch gekennzeichnet, daß man die Behandlung der Säure I, des Hydrats des Äthanolsolvats, des Ameisensäuresolvats oder des Gemisches des Hydrats und des Äthanol- oder Ameisensäuresolvats dieser Säure mit dem Natriumsalz II in wäßrigem Methanol bei einer Temperatur von ungefähr 0°C bewirkt.

4. Verfahren gemäß Anspruch 2 zur Herstellung des in Anspruch 1 definierten kristallinen hydratisierten Salzes, dadurch gekennzeichnet, daß man die Säure der Formel I, ein Hydrat, ein Äthanol- oder Ameisensäuresolvat oder ein Gemisch des Hydrats und des Äthanol- oder Ameisensäuresolvats dieser Säure mit einem Natriumsalz einer organischen Säure in Methanol behandelt, das kristalline Methanolsolvat des erhaltenen Natriumsalzes isoliert, dieses Methanolsolvat ind einem mit Wasser mischbaren und eine geringe Menge Wasser enthaltenden Lösungsmittel suspendiert, in Suspension beläßt und anschließend die gewünschte kristalline Form D isoliert.

5. Verfahren gemäß einem der Ansprüche 2 bis 4, dadurch gekennzeichnet daß das Natriumsalz der organischen Säure, das man verwendet, ausgewählt wird unter Natriumacetat, Natriumäthylhexanoat und Natriumdiäthylacetat.

6. Verfahren gemäß einem der Ansprüche 3, 4 oder 5, dadurch gekennzeichnet, daß das Lösungsmittel, in dem man zur Erzielung der kristallinen Form D, das Methanolsolvat suspendiert oder mit dem man die Methanollösung des Natriumsalzes der Säure I mischt, Äthanol mit einem Gehalt von 0,5 bis 10% Wasser ist.

7. Pharmazeutische Zusammensetzungen, dadurch gekennzeichnet, daß sie als Wirkstoff in der mit D bezeichneten Form das kristalline hydratisierte Natriumsalz der 3-Acetoxymethyl-7-[2-(2-amino-4-thiazolyl)-2-methoxyiminoacetamido]-ceph-3-em-4-carbonsäure als syn-Isomeres enthalten.

8. Arzneimittel, dadurch gekennzeichnet, daß es aus dem in der mit D bezeichneten kristallinen hydratisierten Form vorliegenden Natriumsalz der 3-Acetoxymethyl-7-[2-(2-amino-4-thiazolyl)-2-methoxyiminoacetamido]-ceph-3-em-4-carbonsäure als syn-Isomerem besteht.

## Claims

1. Hydrated crystalline form, called form "D", of the compound with the formula:

*syn* isomer

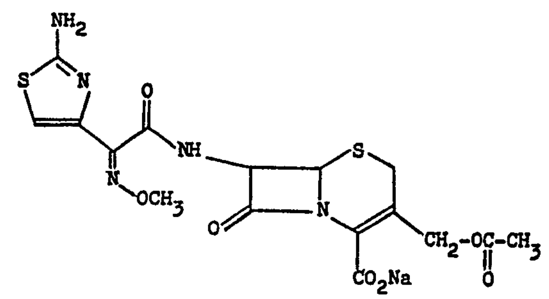

which form has the following powder X-ray diffraction pattern, a pattern obtained with Kα radiation of copper at a wavelength of $\lambda = 1.54$ Å, in which pattern d represents the inter-lattice spacings and $I/I_1$ the relative intensities

| d | $I/I_1$ |
|---|---|
| 9.3 | 0.95 |
| 8.8 | 0.09 |
| 6.5 | 0.07 |
| 6.46 | 0.06 |
| 6.10 | 0.13 |
| 5.30 | 0.25 |
| 5.12 | 0.05 |
| 5.03 | 0.04 |
| 4.57 | 0.04 |
| 4.40 | 0.17 |
| 4.20 | 0.29 |
| 3.96 | 0.70 |
| 3.88 | 0.18 |
| 3.76 | 0.34 |
| 3.6 | 1.00 |
| 3.41 | 0.15 |
| 3.27 | 0.09 |
| 3.19 | 0.11 |
| 3.13 | 0.13 |
| 3.10 | 0.13 |
| 3.03 | 0.15 |
| 2.97 | 0.06 |
| 2.78 | 0.07 |
| 2.72 | 0.04 |
| 2.69 | 0.04 |
| 2.60 | 0.19 |
| 2.50 | 0.11 |
| 2.44 | 0.05 |
| 2.40 | 0.08 |
| 2.32 | 0.09 |
| 2.25 | 0.05 |

and which has the infra-red spectrum in nujol represented in the attached Fig. 1.

2. Process for the preparation of the hydrated crystalline form defined in Claim 1, characterized in that

A) Either the acid of formula I:

syn isomer 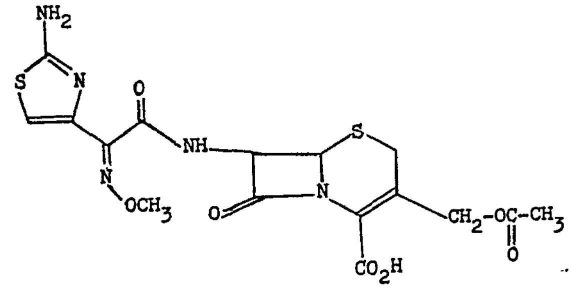 (I)

a hydrate, an ethanol solvate, a formic solvate or a mixture of hydrate and ethanol or formic solvate of this acid is treated with a sodium salt of formula (II)

Na X (II)

in which X represents the anion of an acid of acidity comparable to or less than that of the acid I and the hydrated salt of formula (III) obtained *in situ*

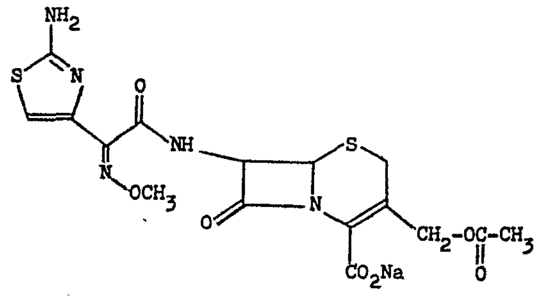 (III)

is crystallized in the presence, if necessary, of an excess of an organic solvent in which the sodium salt is practically insoluble;

B) or the acid of formula (I), a hydrate, an ethanol or formic solvate or a mixture of hydrate and of ethanol or formic solvate of this acid is made to react with a sodium salt of an organic acid in methanol, the crystalline sodium salt of formula (III) in the form of a methanol solvate is isolated, which solvate is converted, if desired, into a product with the formula (III) no longer containing methanol and the product of formula (III) or its methanol solvate is hydrated.

3. Process according to Claim 2, characterized in that the treatment of the acid I, of the hydrate, of the ethanol solvate, of the formic solvate or of the mixture of hydrate and ethanol or formic solvate of this acid with the sodium salt II is carried out in aqueous methanol at a temperature of about 0°C.

4. Process according to Claim 2 for the preparation of the hydrated crystalline salt defined in Claim 1, characterized in that the acid of formula (I), a hydrate, an ethanol or formic solvate or a mixture of hydrate and of ethanol or formic solvate of this acid is treated with a sodium salt of an organic acid in methanol, the crystalline methanol solvate of the sodium salt obtained is isolated, this methanol solvate is put into suspension in a solvent miscible with water and containing a small amount of water, it is left in suspension, when the crystalline form D sought is isolated.

5. Process according to any one of Claims 2 to 4, characterized in that the sodium salt of an organic acid which is used is selected from the group formed by sodium acetate, sodium ethyl hexanoate and sodium diethyl acetate.

6. Process according to any one of Claims 3, 4 or 5, characterized in that the solvent, in which the methanol solvate is suspended or with which the methanol solution of the sodium salt of the acid I is mixed to obtain the crystalline form D, is ethanol containing from 0.5 to 10% of water.

7. Pharmaceutical compositions, characterized in that they contain, as active principle, the sodium salt in hydrated crystalline form, called form D, of 3-acetoxymethyl 7-[2-(2-amino 4-thiazolyl) 2-methoxyimino acetamido] ceph-3-eme 4-carboxylic acid, *syn* isomer.

8. A medicament, the sodium salt in hydrated crystalline form, called form D, of 3-acetoxymethyl 7-[2-(2-amino 4-thiazolyl) 2-methoxyimino acetamido] ceph-3-eme 4-carboxylic acid, *syn* isomer.

FIGURE I